# EUROPÄISCHE PATENTSCHRIFT

(11) **EP 0 934 253 B1**
(45) Veröffentlichungstag und Bekanntmachung des Hinweises auf die Patenterteilung: **27.03.2002**
(21) Anmeldenummer: 97912101.9
(22) Anmeldetag: 02.10.1997
(51) Int. Cl.: C07C 251/52, A01N 37/50

(54) **HALOGENALKOXIMINOESSIGSÄUREAMIDE**
HALOGENALKOXIMINOACETAMIDE
HALOGENALKOXIMINOACETAMIDE

(30) Priorität: 15.10.1996 DE 19642530
(43) Veröffentlichungstag der Anmeldung: 11.08.1999
(73) Patentinhaber: BAYER AG, 51368 Leverkusen (DE)
(72) Erfinder: SEITZ, Thomas, D-40764 Langenfeld (DE); STENZEL, Klaus, D-70595 Düsseldorf (DE)
(86) Internationale Anmeldenummer: EP9705431
(87) Internationale Veröffentlichungsnummer: WO9816500

(56) Entgegenhaltungen:
- EP-A- 0 135 142
- EP-A- 0 153 229
- WO-A-96/06072
- WO-A-96/10556
- DE-A- 4 424 788
- FR-A- 2 515 182

## Beschreibung

Die Erfindung betrifft neue Halogenalkoximinoessigsäureamide, ein Verfahren zu ihrer Herstellung und ihre Verwendung als Schädlingsbekämpfungsmittel.

In EP-A2-153 299 werden neue Cephalosporinderivate beschrieben.

In EP-A2-135 142 werden Verfahren zur Herstellung von Cephemderivaten beschrieben. In FR-A-2515 182 werden Derivate der 3-Amino-2-oxoazetidinsulfonsäure beschrieben.

Es wurden neue Verbindungen der allgemeinen Formel (I) gefunden, 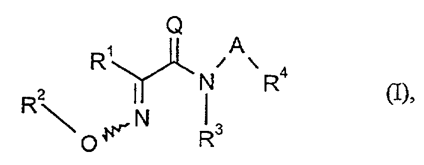 in welcher
- A: für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy,
- R²: für Fluormethyl, Difluormethyl, Trifluormethyl, Difluorchlormethyl, Trifluorethyl, Trifluorethenyl, Dichlorethenyl, Dichlorallyl, Iodpropargyl steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Schließlich wurde gefunden, daß die neuen Halogenalkoximinoessigsäureamide der allgemeinen Formel (I) sehr starke fungizide Wirkung zeigen.

Die erfindungsgemäßen Verbindungen liegen gegebenenfalls als Mischungen verschiedener möglicher isomerer Formen, insbesondere von Stereoisomeren, wie z. B. E-und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beschrieben und beansprucht.

Besonders bevorzugt sind Verbindungen der Formel (I), in welcher
- A: für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
- R²: für Fluormethyl, Difluormethyl, Trifluormethyl, Difluorchlormethyl, Trifluorethyl steht,
- R³: für Wasserstoff oder für Methyl steht,
- R⁴: für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I), in welcher
- A: für eine Einfachbindung oder für Methylen, 1,1-Ethylen, 1,2-Ethylen, 1,1-Propylen, 1,2-Propylen, 1,3-Propylen oder 2,2-Propylen steht,
- Q: für Sauerstoff steht,
- R¹: für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
- R²: für Fluormethyl, Difluormethyl, Trifluormethyl, Difluorchlormethyl, Trifluorethyl steht,
- R³: für Wasserstoff oder Methyl steht,
- R⁴: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Von ganz besonderem Interesse sind Verbindungen der Formel (I), in welcher der Rest R¹ für unsubstituiertes oder in 3- und/oder 4-Stellung substituiertes Phenyl, oder für unsubstituiertes oder in 4- und/oder 5-Stellung substituiertes Furanyl oder Thienyl steht, wobei als Substituenten die oben genannten und insbesondere Chlor, Brom, Fluor, Nitro, Methylsulfonyl, Phenyl, Phenoxy, Benzyloxy, Cyclopropyl, Cyclohexyl, Methyl, Ethyl, n-, i-Propyl, n-, i-, s-, t-Butyl, n-Pentyl, n-Hexyl, n Heptyl, Methoxy, Ethoxy, Methylthio, Trifluormethyl und Trifluormethoxy ausgewählt sind oder jeweils gegebenenfalls durch Fluor substituiertes 3,4-Methylen- und Ethylendioxo, Propan-1,3-diyl und Butan-1,4-diyl substituiertes Phenyl oder in 2-Stellung substituiertes Naphthyl, Benzofuranyl oder Benzothienyl bedeuten.

Insbesondere sind auch Verbindungen der Formel (I) bevorzugt, in denen
- R³: für Wasserstoff steht.

Insbesondere sind auch Verbindungen der Formel (I) bevorzugt, in denen R⁴ für in 3-und 4-Stellung durch Methoxy substituiertes Phenyl steht.

In einer weiterhin besonders bevorzugten Gruppe von Verbindungen steht A für -CH₂-CH₂-.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer Vorzugsbereiche ersetzt.

Bevorzugte Einzelverbindungen können den folgenden Tabellen entnommen werden:

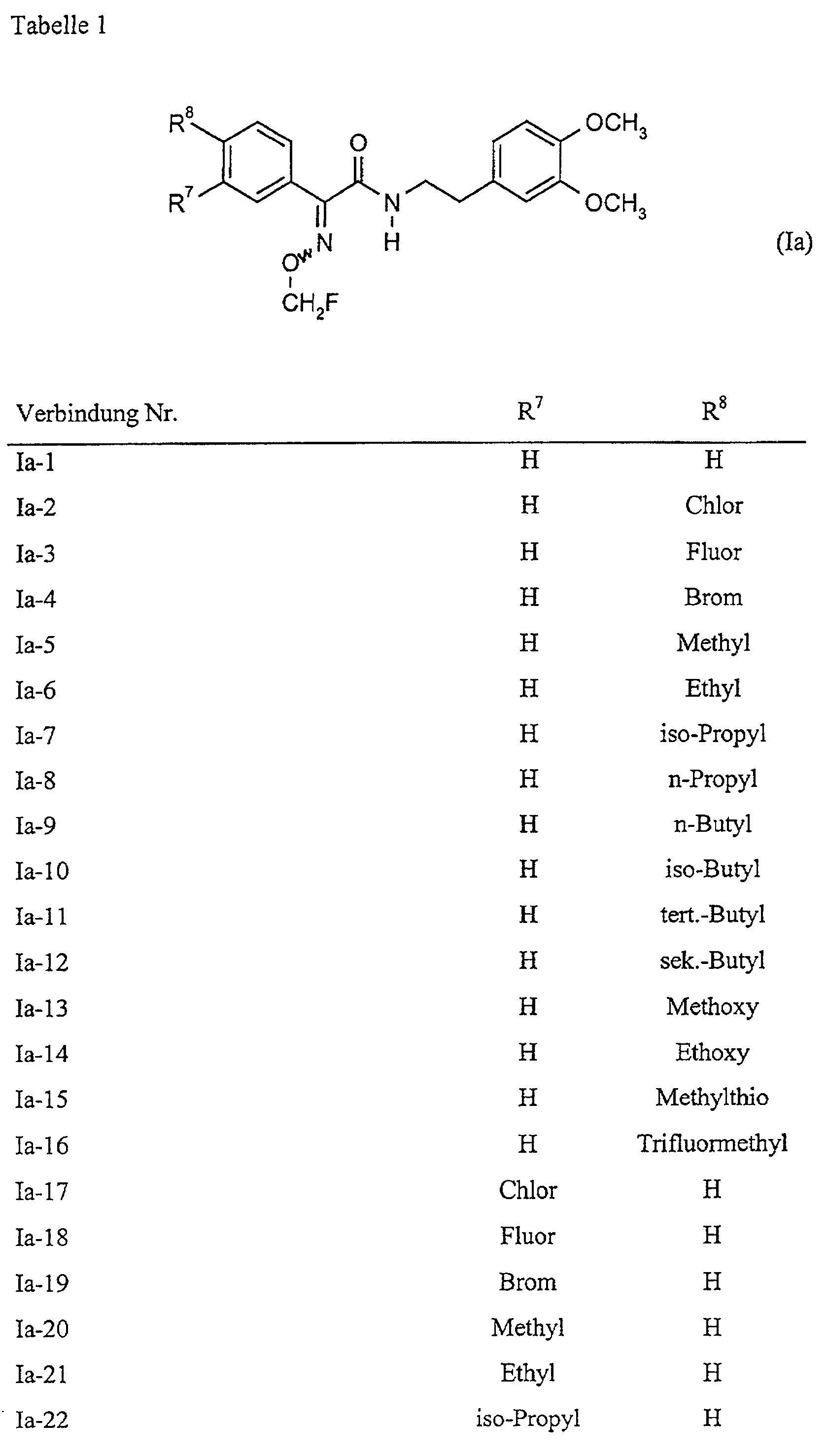

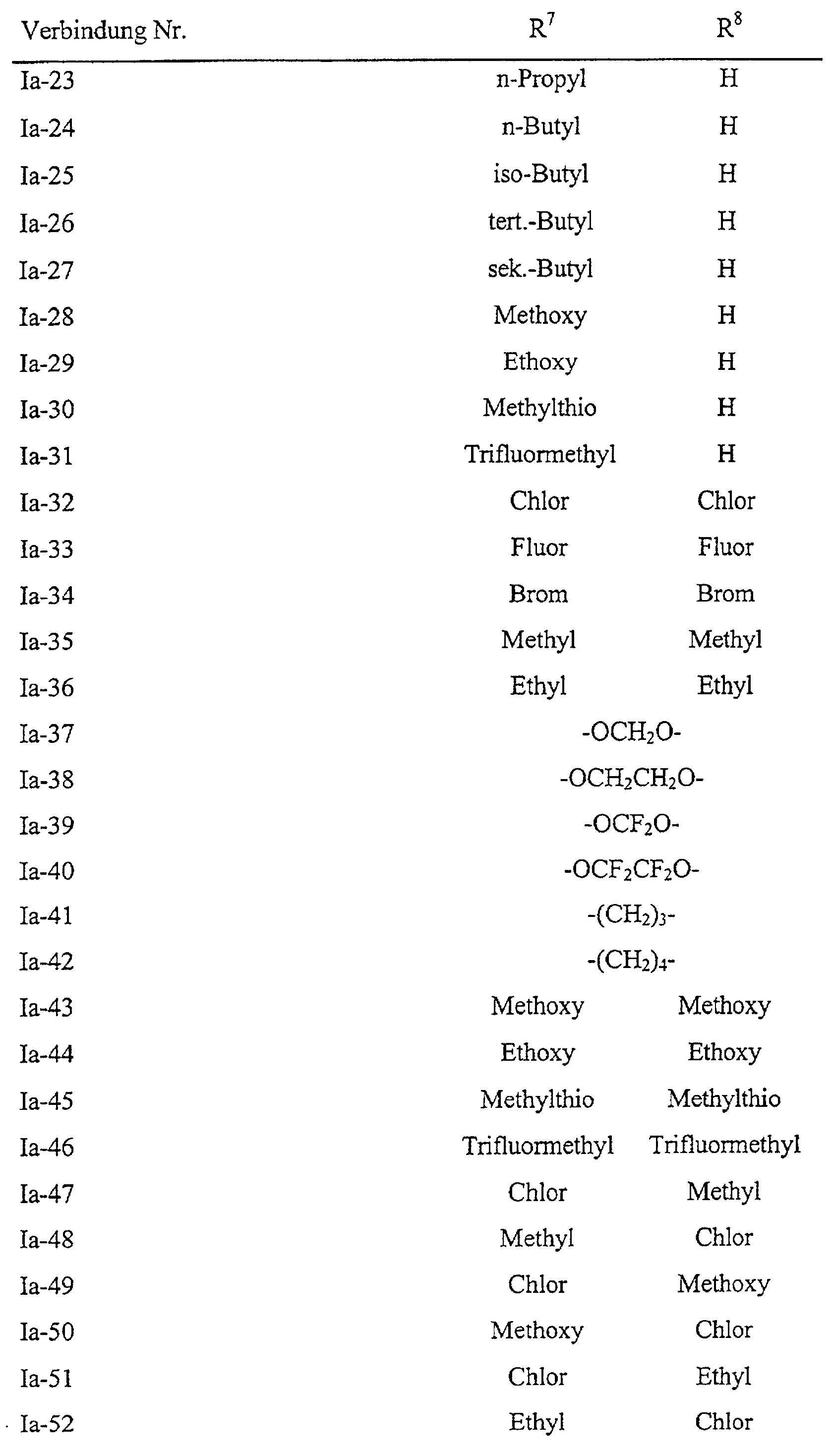

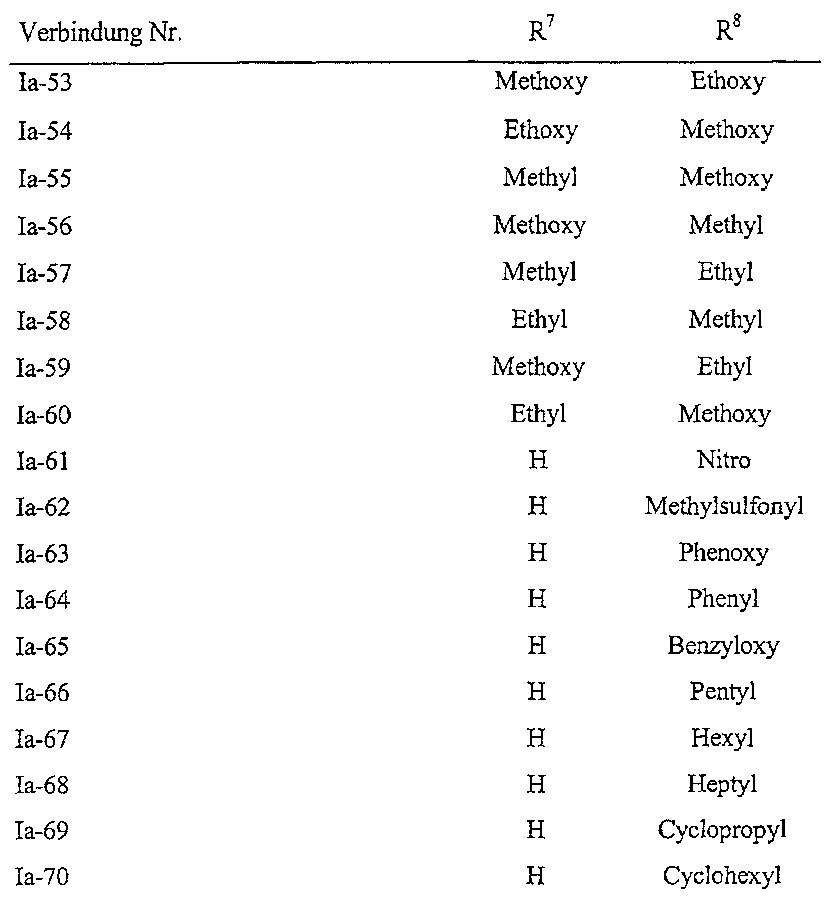

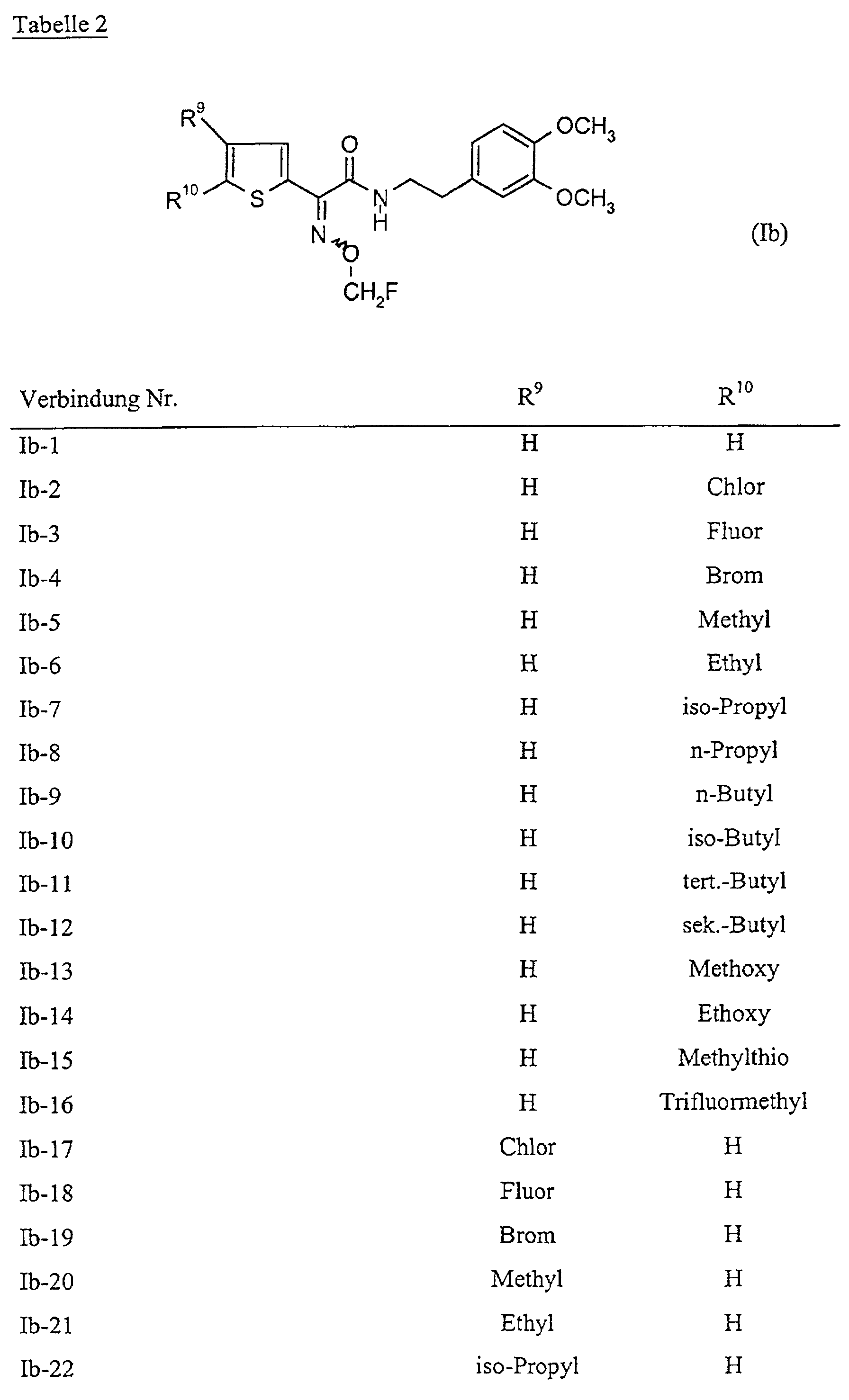

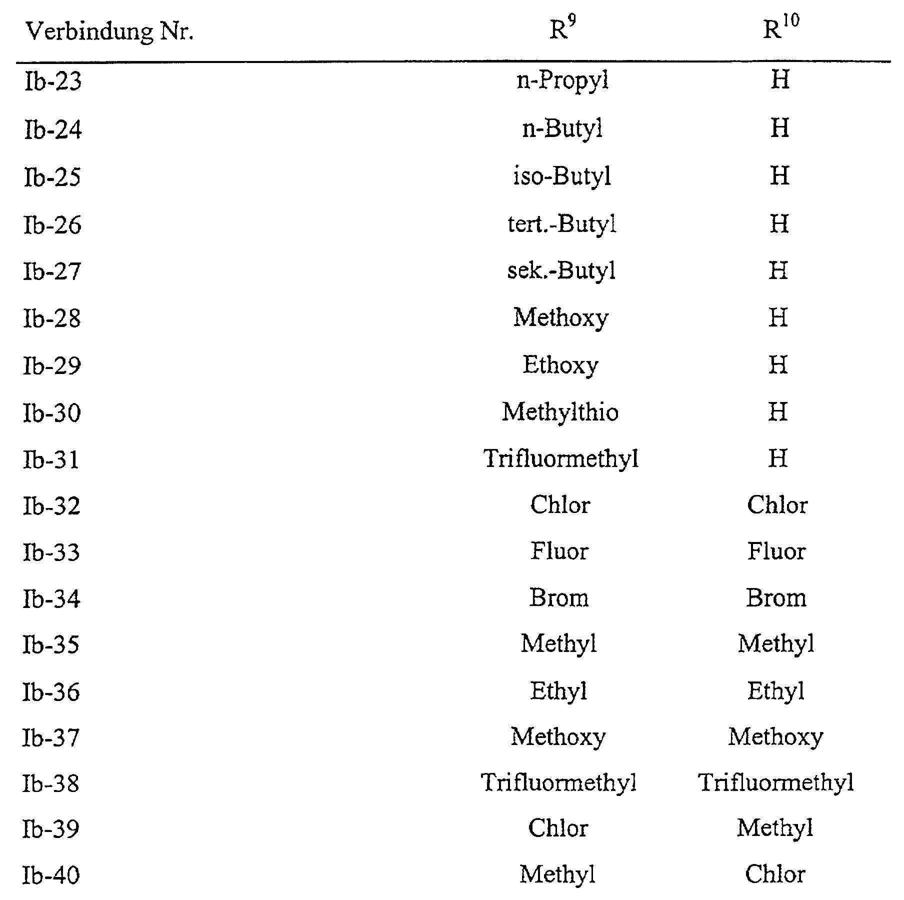

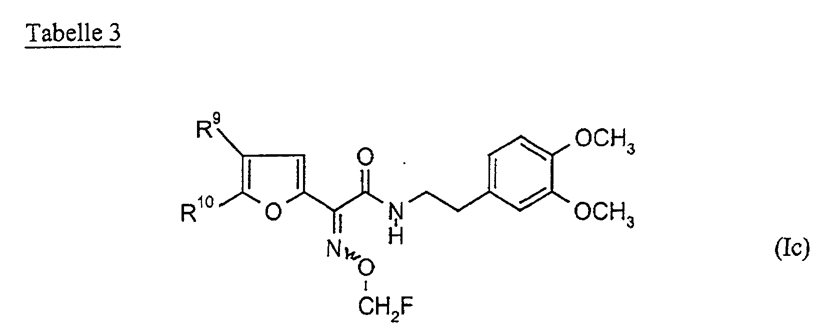

wobei R⁹ und R¹⁰ für die in Tabelle 2 genannten Substituenten stehen.

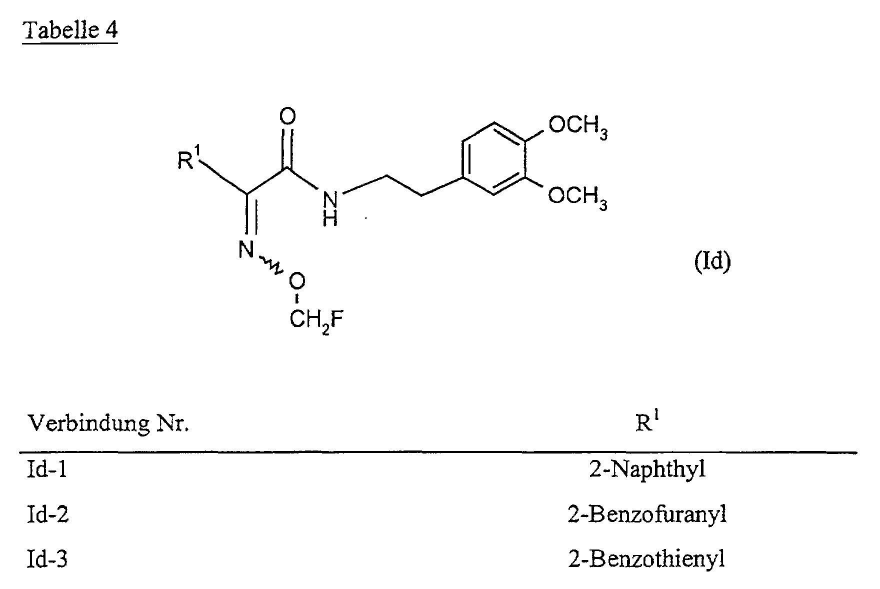

### Tabelle 5

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁴) ein Phenylrest steht, der die in den Verbindungen Ia-1 bis Ia-70 als R⁷ und R⁸ jeweils angegebenen Substituenten trägt.

### Tabelle 6

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁴) einer der folgenden dreifach substituierten Phenylreste steht: 3,4,5-Trimethoxyphenyl; 3,4,5-Trichlorphenyl; 3,4,5-Trimethylphenyl.

### Tabelle 7

Verbindungen Ia-1 bis Id-3 entsprechend den Formeln Ia, Ib, Ic und Id, wobei anstelle der Fluormethylgruppe-Gruppe (im allgemeinen bezeichnet als R²) eine Difluormethyl-Gruppe steht.

### Tabelle 8

Verbindungen Ia-1 bis Id-3 entsprechend den Formeln Ia, Ib, Ic und Id, wobei anstelle der Fluormethylgruppe-Gruppe (im allgemeinen bezeichnet als R²) eine Trifluormethyl-Gruppe steht.

Weiterhin wurde gefunden, daß man die neuen Halogenalkoximinoessigsäureamide der allgemeinen Formel (I) erhält, wenn man Glyoxylsäureoxime der Formel (II) 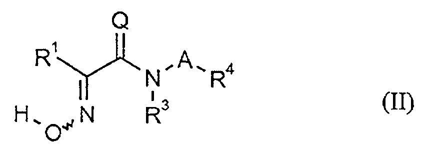 in welcher
- A, R¹, R³ und R⁴: die oben angegebenen Bedeutungen haben, mit einer Halogenverbindung der Formel (III),

R²-X
in welcher
- R²: die oben angegebene Bedeutung hat und
- X: für Halogen steht,
gegebenenfalls in Gegenwart eines Verdünnungsmittels und gegebenenfalls in Gegenwart einer Base, umsetzt.

Die zur Durchführung des erfindungsgemäßen Verfahrens als Ausgangsstoffe benötigten Glyoxylsäureoxime sind durch die Formel (II) allgemein definiert. In dieser Formel (II) haben A, R¹, R³ und R⁴ die vorzugsweise bzw. insbesondere diejenige Bedeutungen, die bereits im Zusammenhang mit der Beschreibung der erfindungsgemäßen Verbindungen der Formel (I) als bevorzugt bzw. als insbesondere bevorzugt für A, R¹, R³ und R⁴ angegeben wurden.

Die Ausgangsstoffe der Formel (II) werden erhalten, wenn man Carbonsäurederivate der allgemeinen Formel (IV) 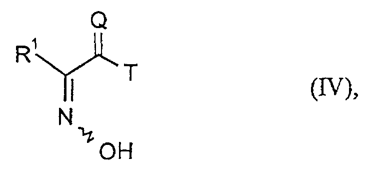 in welcher
- R¹ und Q: die oben angegebenen Bedeutungen haben und
- T: für Hydroxy, Halogen oder Alkoxy steht,
mit einem Amin der allgemeinen Formel (V) 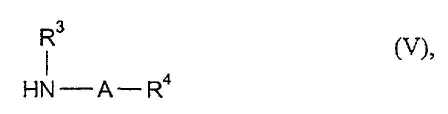 in welcher
- R³, R⁴ und A: die oben angegebenen Bedeutungen haben,
- oder mit einem Hydrogenhalogenid hiervon -
gegebenenfalls in Gegenwart eines Säureakzeptors, wie beispielsweise Triethylamin, gegebenenfalls in Gegenwart eines Kondensationsmittels, wie beispielsweise Dicyclohexylcarbodiimid, und gegebenenfalls in Gegenwart eines Verdünnungsmittels, wie beispielsweise Toluol, umsetzt.

Die zur Herstellung der Glyoxylsäureoxime der Formel (II) als Ausgangsstoffe benötigten Carbonsäurederivate sind durch die Formel (IV) allgemein definiert. In dieser Formel (IV) haben Q und R¹ vorzugsweise, bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Q und R¹ angegeben wurden; T steht vorzugsweise für Alkoxy mit 1 bis 4 Kohlenstoffatomen, insbesondere für Methoxy oder Ethoxy, für Hydroxy oder Chlor.

Die Ausgangsstoffe der Formel (IV) sind bekannt und/oder können nach an sich bekannten Verfahren hergestellt werden (vgl. EP-A 178 826, EP-A 242 081, EP-A 382 375, EP-A 493 711, EP-A 432 503, DE-A 3 938 054).

Die weiterhin zur Herstellung der Glyoxylsäureoxime der Formel (II) als Ausgangsstoffe benötigten Amine sind durch die Formel (V) allgemein definiert. In dieser Formel (V) haben R³, R⁴ und A vorzugsweise, bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R³, R⁴ und A angegeben wurden.

Die Amine der Formel (V) sind bekannte organische Synthesechemikalien und/oder können nach an sich bekannten Verfahren hergestellt werden.

Die weiterhin zur Durchführung des erfindungsgemäßen Verfahrens zur Herstellung der erfindungsgemäßen Verbindungen der Formel (I) benötigten Halogenverbindungen sind durch die Formeln (III) allgemein definiert. In dieser Formeln (III) hat R² vorzugsweise, bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R² angegeben wurde.

Die Halogenverbindungen der Formel (III) sind bekannte organische Synthesechemikalien und/oder können nach an sich bekannten Verfahren hergestellt werden.

Als Verdünnungsmittel zur Durchführung des erfindungsgemäßen Verfahrens kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; oder Sulfone, wie Sulfolan.

Das erfindungsgemäße Verfahren wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen von -30°C bis 150°C, vorzugsweise bei Temperaturen von -10°C bis 80°C.

Zur Durchführung des erfindungsgemäßen Verfahrens zur Herstellung der Verbindungen der Formel (I) setzt man pro Mol des Glyoxylsäureoxims der Formel (II) im allgemeinen 1 bis 15 Mol, vorzugsweise 2 bis 8 Mol Halogenverbindung der Formel (III) ein.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vergleiche auch die Herstellungsbeispiele).

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara-Arten eingesetzt. Mit gutem Erfolg werden auch Krankheiten im Getreidebau, wie beispielsweise Septoria-Arten bekämpft. Ferner lassen sich die erfindungsgemäßen Verbindungen auch zur Steigerung des Ernteertrags von Kulturpflanzen einsetzen

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
   Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilate, Iminoctadinetriacetate, Iodocarb, Ipconazol, Iprobenfos(IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Boerdeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Zirarn sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-β-propyl-1H-1,2,4-triazol-1-ethanol,
   α-(2,4-Dichlorphenyl)-ß-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-α-(Methoxyimino)-N-methyl-2-phenoxy-phenylacetamid,
   {2-Methyl-1-[[[1-(4-methylphenyl)ethyl]amino]carbonyl]propyl}-carbaminsäure-1-isopropylester
   1-(2,4-Dichlorphenyl)-2-(1H-1,2,4-triazol-1-yl)ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolari-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)methoxy]phenyl]ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)phenyl]methyl]-benzamid,
   2-(2,3,3-Triiod-2-propenyl)-2H-tetrazol,
   2-[(1-Methylethyl)sulfonyl]-5-(trichlormethyl)-1,3,4-thiadiazol,
   2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)oxy]methyl]-benzamid,
   3-(1,1-Dimethylpropyl-1-oxo-lH-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol, -Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis(2-propinyloxy)-phenyl]-N'-methoxy-methanimidamid,
   N-Formyl-N-hydroxy-DL-alanin, -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin,
   Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

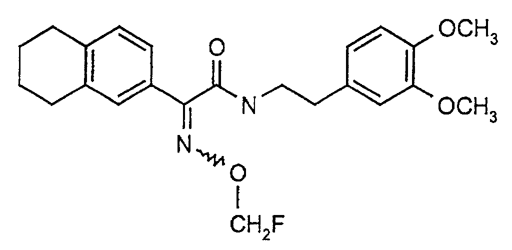

Eine Lösung von 1,91g (5 mMol) N-[(3,4-Dimethoxyphenyl)-2-ethyl]-2-hydroxyimino-2-(2-tetrahydronaphthyl) essigsäureamid in 20 ml Diethylether wird zu 0,2 g (6 mMol) 80%iger Natriumhydridsuspension getropft. Nach Ende der Gasentwicklung tropft man eine Lösung von 0,8 g (7 mMol) Bromfluormethan in 5 ml Diethylether bei -5°C zu und läßt 2 Stunden bei 0°C rühren. Nachdem sich das Gemisch auf Raumtemperatur erwärmt hat, wird mehrmals mit Wasser gewaschen, die organische Phase über Natriumsulfat getrocknet und das Lösungsmittel bei vermindertem Druck abdestilliert.

Man erhält 0,3 g (15 % der Theorie) N-[(3,4-Dimethoxyphenyl)-2-ethyl]-2-fluormetoxyimino-2-(2-tetrahydronaphthyl)-essigsäureamid als farbloses Öl.
MS: m/e = 414 (M⁺)

### Herstellung des Ausgangsstoffes

### Beispiel II-1

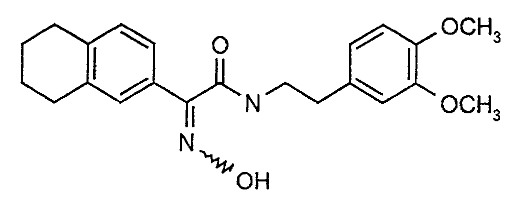

16,8g (0,0679 mol) 2-Hydroximino-2-(2-tetrahydronaphthyl)-essigsäureester, 12,3 g (0,0679 mol) 2-(3,4-Dimethoxyphenyl)-ethylamin und 24,4g (0,136 mol) 30%-ige Natriummethylatlösung werden in 100 ml Methanol 16 Stunden unter Rückfluß zum Sieden erhitzt. Nach Abdestillieren des Lösungsmittels wird der Rückstand in Dichlormethan aufgenommen und mehrmals mit Wasser gewaschen. Die organische Phase wird über Natriumsulfat getrocknet und das Lösungsmittel bei vermindertem Druck abdestilliert.

Man erhält 21,4g (82,3 % der Theorie) N-[(3,4-Dimethoxyphenyl)-2-ethyl]-2-hydroxyimino-2-(2-tetrahydronaphthyl)-essigsäureamid als Feststoff mit einem Schmelzpunkt von 104°C.

Analog Beispiel 1 sowie entsprechend der allgemeinen Beschreibung des erfindungsgemäßen Herstellungsverfahrens, können beispielsweise auch die in der nachstehenden Tabelle 9 aufgeführten Verbindungen hergestellt werden:

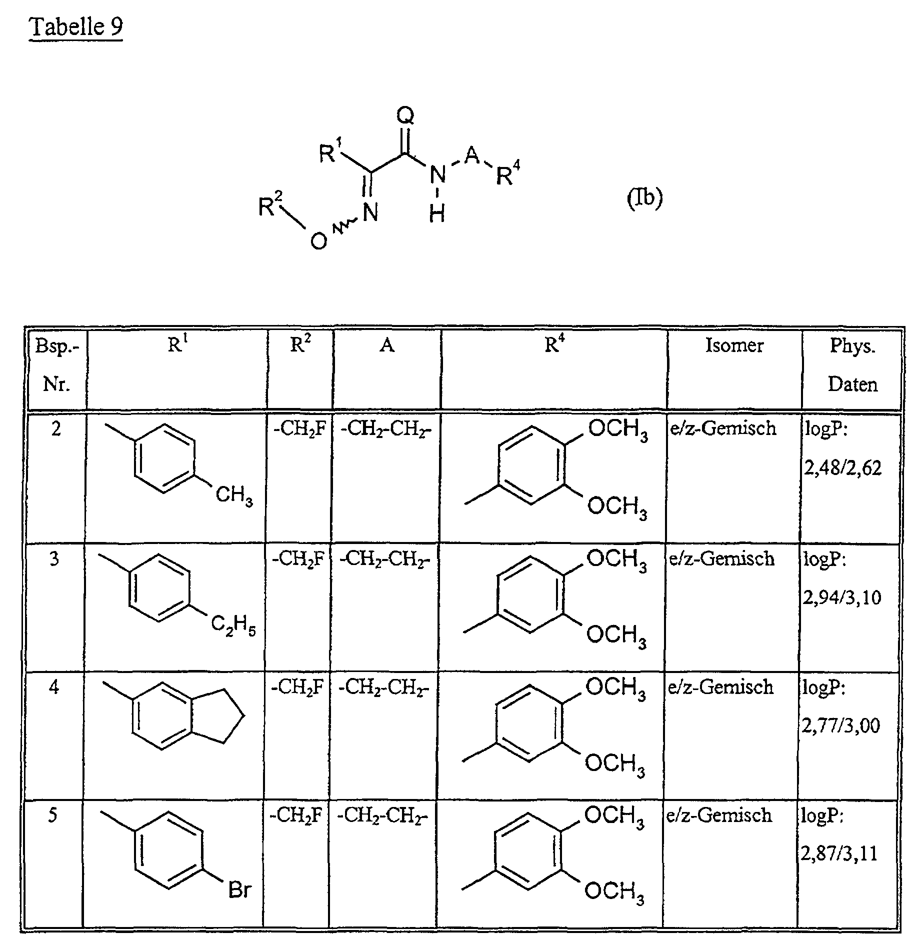

### Anwendungsbeispiele

### Beispiel A

| Plasmopara-Test (Reben) / protektiv | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei 20°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Bei diesem Test zeigen z.B. die folgenden Verbindungen der Herstellungsbeispiele (1), (2), (3) und (4) bei einer beispielhaften Wirkstoffaufwandmenge von 10 g/ha einen Wirkungsgrad bis zu 94 % im Vergleich zur unbehandelten Kontrolle.

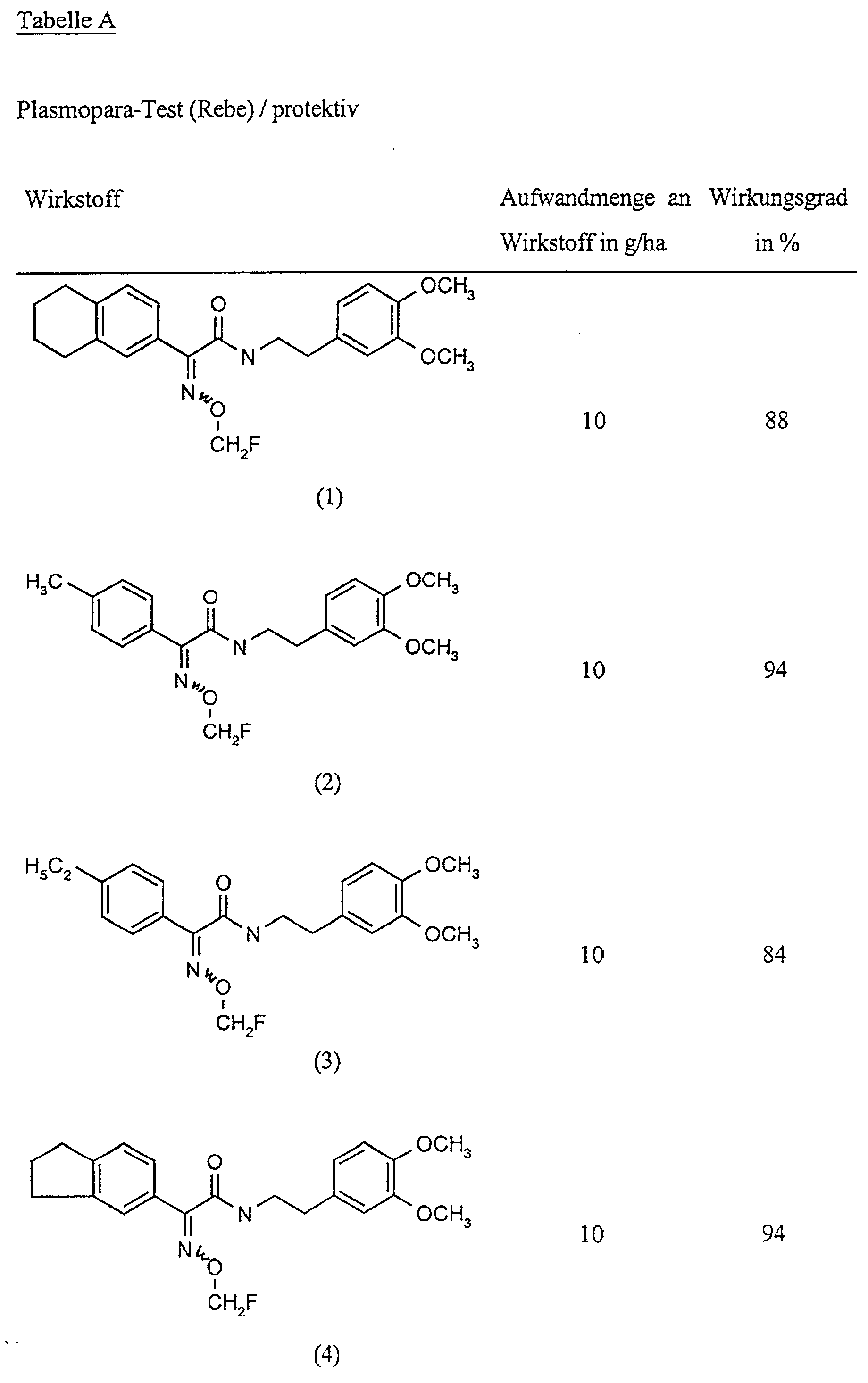

## Patentansprüche

1. Verbindungen der Formel (I), in welcher 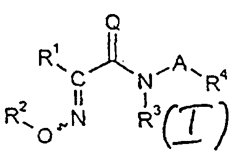
A für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n-oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy,
R² für Fluormethyl, Difluormethyl, Trifluormethyl, Difluorchlormethyl, Trifluorethyl,
Trifluorethenyl, Dichlorethenyl, Dichlorallyl
Iodpropargyl steht,
R³ für Wasserstoff oder für Methyl oder Ethyl steht,
R⁴ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

2. Verbindungen der Formel (I) gemäß Anspruch 1, in welcher
A für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3-oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
R² für Fluormethyl, Difluormethyl, Trifluormethyl, Difluorchlormethyl, Trifluorethyl steht,
R³ für Wasserstoff oder für Methyl steht,
R⁴ für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

3. Verbindungen der Formel (I) gemäß Anspruch 1, in welcher
A für eine Einfachbindung oder für Methylen, 1,1-Ethylen, 1,2-Ethylen, 1,1-Propylen, 1,2-Propylen, 1,3-Propylen oder 2,2-Propylen steht,
Q für Sauerstoff steht,
R¹ für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
R² für Fluormethyl, Difluormethyl, Trifluormethyl, Difluorchlormethyl, Trifluorethyl steht,
R³ für Wasserstoff oder Methyl steht,
R⁴ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

4. Schädlingsbekämpfungsmittel, **gekennzeichnet durch** einen Gehalt an mindestens einer Verbindung der Formel (I) nach Anspruch 1.

5. Verfahren zur Bekämpfung von Schädlingen, **dadurch gekennzeichnet, daß** man Verbindungen der Formel (I) nach Anspruch 1 auf Schädlinge und/ oder ihren Lebensraum einwirken läßt.

6. Verfahren zur Herstellung von Verbindungen der Formel (I), **dadurch gekennzeichnet, daß** man Glyoxylsäureoxime der Formel (II) 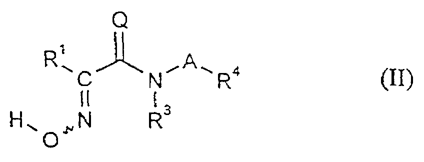 in welcher
A, R¹, R³ und R⁴ die in Anspruch 1 angegebenen Bedeutungen haben, mit einer Halogenverbindung der Formel (III),
R²-X
in welcher
R² die oben angegebene Bedeutung hat und
X für Halogen steht,
gegebenenfalls in Gegenwart eines Verdünnungsmittels und gegebenenfalls in Gegenwart einer Base, umsetzt.

7. Verwendung von Verbindungen der Formel (I) nach den Ansprüchen 1 bis 3 zur Bekämpfung von Schädlingen.

8. Verfahren zur Herstellung von Schädlingsbekämpfungsmitteln, **dadurch gekennzeichnet, daß** man Verbindungen der Formel (I) nach den Ansprüchen 1 bis 3 mit Streckmitteln und/oder oberflächenaktiven Mitteln vermischt.

## Claims

1. Compounds of the formula 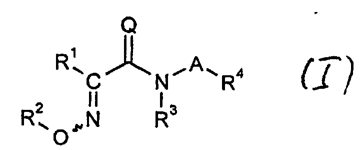 in which
A represents a single bond or represents methylene, 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents selected from the group consisting of fluorine, chlorine, cyano and methoxy,
Q represents oxygen or sulphur,
R¹ represents in each case optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, where the possible substituents are preferably selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, n-pentyl, n-hexyl, n-heptyl, methoxy, ethoxy, nor i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- or i-propyl;
cyclopropyl, cyclobutyl, cyclopentyl or cyclohexyl;
benzyl, phenoxy, benzyloxy, phenyl, optionally substituted by the substituents mentioned above,
R² represents fluoromethyl, difluoromethyl, trifluoromethyl, difluorochloromethyl, trifluoroethyl,
trifluoroethenyl, dichloroethenyl, dichlorallyl
iodopropargyl,
R³ represents hydrogen or represents methyl or ethyl,
R⁴ represents in each case optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, where the possible substituents are preferably selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical substituents selected from the group consisting of fluorine, chlorine, methyl, trifluoromethyl, ethyl, n- or i-propyl,
cyclopropyl, cyclobutyl, cyclopentyl or cyclohexyl.

2. Compounds of the formula (I) according to Claim 1, in which
A represents a single bond or represents 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene) or methylene, in each case optionally mono- or polysubstituted by identical or different substituents selected from the group consisting of fluorine, chlorine, cyano, methoxy,
Q represents oxygen or sulphur,
R¹ represents in each case optionally mono- to hexasubstituted cyclobutyl, cyclopentyl or cyclohexyl, preferred substituents being those mentioned below;
represents in each case optionally mono- to trisubstituted phenyl, naphthyl, furyl, benzofuranyl, thienyl, benzothienyl, pyridyl, quinolyl, tetrahydrofuryl or perhydropyranyl, where the possible substituents are preferably selected from the list below:
fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- or i-propyl; cyclopropyl, cyclopentyl, cyclohexyl; phenyl, phenoxy, benzyloxy, each of which is optionally substituted by the abovementioned substituents;
R² represents fluoromethyl, difluoromethyl, trifluoromethyl, difluorochloromethyl, trifluoroethyl,
R³ represents hydrogen or represents methyl,
R⁴ represents in each case optionally mono- to hexasubstituted cyclopropyl, cyclobutyl, cyclopentyl or cyclohexyl, where the preferred substituents are those mentioned below;
represents in each case optionally mono- to trisubstituted phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, where the possible substituents are preferably selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl, in each case doubly attached trimethylene (propane-1,3-diyl), tetramethylene (butane-1,4-diyl), methylenedioxy or ethylenedioxy, each of which is optionally mono- or polysubstituted by identical or different substituents selected from the group consisting of fluorine, chlorine, methyl, trifluoromethyl, ethyl, n- and i-propyl,
cyclopropyl, cyclobutyl, cyclopentyl or cyclohexyl.

3. Compounds of the formula (I) according to Claim 1, in which
A represents a single bond or represents methylene, 1,1-ethylene, 1,2-ethylene, 1,1-propylene, 1,2-propylene, 1,3-propylene or 2,2-propylene,
Q represents oxygen,
R¹ represents thienyl, furanyl or phenyl which is in each case optionally mono- or disubstituted by bromine, chlorine, fluorine, nitro, methylsulphonyl, phenyl, phenyloxy, benzyloxy, cyclopropyl, cyclohexyl, methyl, ethyl, n- or i-propyl, n-, i-, s- or t-butyl, n-pentyl, n-hexyl, n-heptyl, methoxy, ethoxy, trifluoromethyl, trifluoromethoxy and/or methylthio, or represents phenyl which is substituted by in each case optionally fluorine-substituted 3,4-methylene- and ethylenedioxo, propane-1,3-diyl and butane-1,4-diyl or represents naphthyl, benzofuranyl or benzothienyl,
R² represents fluoromethyl, difluoromethyl, trifluoromethyl, difluorochloromethyl, trifluoroethyl,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl, quinolyl, where the possible substituents are preferably selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl,
in each case doubly attached trimethylene (propane-1,3-diyl), tetramethylene (butane-1,4-diyl), methylenedioxy or ethylenedioxy, each of which is optionally mono- or polysubstituted by identical or different substituents selected from the group consisting of fluorine, chlorine, methyl, trifluoromethyl, ethyl, n- and i-propyl.

4. Pesticides, **characterized in that** they contain at least one compound of the formula (I) according to Claim 1.

5. Method for controlling pests, **characterized in that** compounds of the formula (I) according to Claim 1 are allowed to act on pests and/or their habitat.

6. Process for preparing compounds of the formula (I), **characterized in that** glyoxylic acid oximes of the formula (II) 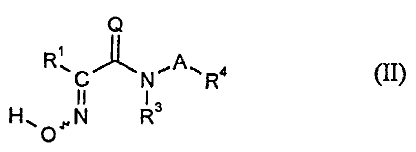 in which
A, R¹, R³ and R⁴ are as defined in Claim 1 are reacted with a halogen compound of the formula (III),
R²-X
in which
R² is as defined above and
X represents halogen,
if appropriate in the presence of a diluent and if appropriate in the presence of a base.

7. The use of compounds of the formula (I) according to Claims 1 to 3 for controlling pests.

8. Process for preparing pesticides, **characterized in that** compounds of the formula (I) according to Claims 1 to 3 are mixed with extenders and/or surfactants.

## Revendications

1. Composés de formule (I) 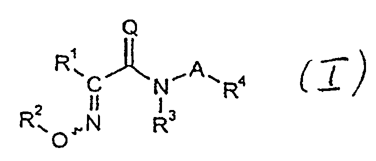 dans laquelle
A est une liaison simple ou représente un groupe méthylène, 1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène), chacun étant substitué éventuellement une ou plusieurs fois identiques ou différentes par du fluor, du chlore, un radical cyano ou méthoxy,
Q représente l'oxygène ou le soufre,
R¹ représente un groupe cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle dont chacun porte, le cas échéant, un à trois substituants, les substituants possibles étant choisis de préférence parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants, identiques ou différents, fluoro, chloro, méthyle, trifluorométhyle, éthyle, n-propyle ou isopropyle ;
cyclopropyle, cyclobutyle, cyclopentyle ou cyclohexyle ;
phényle, benzyle, phénoxy, benzyloxy portant éventuellement les substituants mentionnés ci-dessus,
R² représente un groupe fluorométhyle, difluorométhyle, trifluorométhyle, difluorochlorométhyle, trifluoréthyle,
trifluoréthényle, dichloréthényle, dichlorallyle, iodopropargyle,
R³ représente l'hydrogène ou un groupe méthyle ou éthyle,
R⁴ représente un groupe, éventuellement substitué une à trois fois dans chaque cas, cyclopropyle, cyclobutyle, cyclopentyle ou cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle, les substituants possibles étant choisis de préférence parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents,
cyclopropyle, cyclobutyle, cyclopentyle ou cyclohexyle.

2. Composés de formule (I) suivant la revendication 1, dans laquelle
A est une liaison simple ou représente un groupe méthylène, 1,1-éthylène, 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène), portant éventuellement un ou plusieurs substituants, identiques ou différents, fluoro, chloro, cyano, méthoxy,
Q est l'oxygène ou le soufre,
R¹ représente un groupe cyclobutyle, cyclopentyle ou cyclohexyle, dont chacun porte éventuellement 1 à 6 substituants, les substituants appréciés étant mentionnés ci-dessous :
phényle, naphtyle, furyle, benzofurannyle, thiényle, benzothiényle, pyridyle, quinolyle, tétrahydrofuryle ou perhydropyrannyle portant chacun, le cas échéant, un à trois substituants, les substituants possibles étant avantageusement choisis parmi ceux qui sont énumérés ci-après :
fluor, chlore, brome, cyano, nitro, méthyle, éthyle, n-propyle, isopropyle, n-butyle, isobutyle, sec.-butyle, tertio-butyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents ;
cyclopropyle, cyclopentyle, cyclo-hexyle ;
phényle, phénoxy, benzyloxy portant éventuellement les substituants mentionnés ci-dessus,
R² est un groupe fluorométhyle, difluorométhyle, trifluorométhyle, difluorochlorométhyle, trifluoréthyle,
R³ est l'hydrogène ou le groupe méthyle,
R⁴ est un groupe cyclopropyle, cyclobutyle, cyclopentyle ou cyclohexyle portant chacun, le cas échéant, un à six substituants, les substituants appréciés étant mentionnés ci-dessous ;
un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle, portant chacun, le cas échéant, un à trois substituants, les substituants possibles étant choisis de préférence parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle, triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents,
cyclopropyle, cyclobutyle, cyclopentyle ou cyclohexyle.

3. Composés de formule (I) suivant la revendication 1, dans laquelle
A est une liaison simple ou représente un groupe méthylène, 1,1-éthylène, 1,2-éthylène, 1,1-propylène, 1,2-propylène, 1,3-propylène ou 2,2-propylène,
Q est l'oxygène,
R¹ représente un groupe phényle, thiényle ou furannyle portant éventuellement un ou deux substituants bromo, chloro, fluoro, nitro, méthylsulfonyle, phényle, phényloxy, benzyloxy, cyclopropyle, cyclohexyle, méthyle, éthyle, n-propyle, isopropyle, n-butyle, isobutyle, sec.-butyle, tertio-butyle, n-pentyle, n-hexyle, n-heptyle, méthoxy, éthoxy, trifluorométhyle, trifluorométhoxy et/ou méthylthio, ou représente un groupe phényle portant un substituant 3,4-méthylène- et éthylène-dioxo, propane-1,3-diyle et butane-1,4-diyle dont chacun est éventuellement substitué par du fluor, ou un groupe naphtyle, benzofurannyle ou benzothiényle,
R² est un groupe fluorométhyle, difluorométhyle, trifluorométhyle, difluorochlorométhyle, trifluoréthyle,
R³ représente l'hydrogène ou le groupe méthyle,
R⁴ est un groupe cyclohexyle ou un groupe, éventuellement substitué une à trois fois, phényle, thiényle, furyle, benzofuryle, benzothiényle, pyridyle, pyrimidinyle, naphtyle, quinolyle, les substituants possibles étant avantageusement choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
un groupe triméthylène (propane-1,3-diyle), tétraméthylène (butane-1,4-diyle), méthylènedioxy ou éthylènedioxy ayant chacun deux liaisons et portant chacun, le cas échéant, un ou plusieurs substituants fluoro, chloro, méthyle, trifluorométhyle, éthyle, n-propyle ou isopropyle identiques ou différents.

4. Compositions pesticides, **caractérisées par** une teneur en au moins un composé de formule (I) suivant la revendication 1.

5. Procédé pour combattre des parasites, **caractérisé en ce qu'**on fait agir des composés de formule (I) suivant la revendication 1 sur les parasites et/ou sur leur milieu.

6. Procédé de production de composés de formule (I), **caractérisé en ce qu'**on fait réagir des oximes d'acide glyoxylique de formule (II) 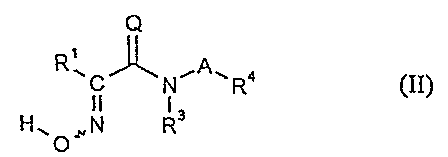 dans laquelle
A, R¹, R³ et R⁴ ont les définitions indiquées dans la revendication 1, avec un composé halogéné de formule (III)
R²-X
dans laquelle
R² a la définition indiquée ci-dessus et
X est un halogène,
le cas échéant en présence d'un diluant et en présence éventuelle d'une base.

7. Utilisation de composés de formule (I) suivant les revendications 1 à 3 pour combattre des parasites.

8. Procédé de préparation de compositions pesticides, **caractérisé en ce qu'**on mélange des composés de formule (I) suivant les revendications 1 à 3 avec des diluants et/ou des agents tensio-actifs.
